# FASCICULE DE BREVET EUROPEEN

(11) **EP 0 521 743 B1**
(45) Date de publication et mention de la délivrance du brevet: **04.02.1998**
(21) Numéro de dépôt: 92401537.3
(22) Date de dépôt: 04.06.1992
(51) Int. Cl.: A61M 1/10

(54) **Procédé et dispositif de commande asservie du moteur d'une prothèse cardiaque péristaltique**
Verfahren und Vorrichtung zum Regeln eines Motors einer peristaltischen Herzprothese
Control system for the motor of a peristaltic cardiac prosthesis

(30) Priorité: 05.06.1991 FR 9106811
(43) Date de publication de la demande: 07.01.1993
(73) Titulaire: Dumas, Jean-Claude, 13008 Marseille (FR); Pol, Vincent, 13008 Marseille (FR); Molina, Jean-Marc, 13127 Vitrolles (FR); Orfimar, 75016 Paris (FR)
(72) Inventeur: Dumas, Jean-Claude, F-13008 Marseille (FR); Pol, Vincent, F-13390 Auriol (FR)
(74) Mandataire: Martin, Jean-Jacques

(56) Documents cités:
- EP-A- 0 148 661
- FR-A- 2 458 288
- IEEE TRANS. BIO. ENGINEERING vol. 37, no. 4, Avril 1990, NEW YORK,U.S. pages 374 - 383; TASCH ET AL: 'AN ADAPTIVE AORTIC PRESSURE OBSERVER FOR THE PENN STATE ELECTRIC VENTRICULAR ASSIST DEVICE'
- IEEE TRANS. ON BIOMED. ENGINEERING vol. 37, no. 12, 12 Décembre 1990, NEW YORK pages 1195 - 1200; MIN ET AL: 'a moving actuator type electromechanical total artificial heart- part II'

## Description

La présente invention concerne d'une façon générale les pompes cardiaques prothétiques, et plus précisément un procédé et un dispositif pour le pilotage asservi d'une pompe cardiaque péristaltique.

On connaît déjà par la demande de brevet européen publiée No. 0 148 661 au nom des demandeurs une pompe sanguine implantable de type péristaltique, qui permet de répondre de manière adaptative à un certain nombre d'exigences physiologiques. Une telle pompe, tout comme le coeur naturel, est une pompe de type péristaltique. Par un choix approprié du point de fonctionnement, sa réponse en débit est linéaire.

Une caractéristique essentielle de cette pompe connue réside en ce que les variations de débit ne dépendent d'aucun capteur extérieur, c'est-à-dire que la pompe, pour fixer son fonctionnement, n'utilise que les informations inhérentes à sa connexion sur le réseau sanguin, c'est-à-dire la pression aortique et le volume du retour veineux. Ainsi une caractéristique du fonctionnement de cette pompe est de ne jamais tendre vers un fonctionnement volumétrique.

Une variation de la pression aortique se produit à chaque fois que l'on observe une vaso-constriction du système périphérique. Ainsi, au cours de la contraction du ventricule artificiel, l'éjection systolique ne peut se produire qu'au moment où la pression dans la poche est devenue au moins égale à la pression aortique.

A la fin de la phase d'éjection, la pression dans la poche sera devenue très supérieure à la pression aortique, et va constituer une réserve d'énergie qu'il est possible d'exploiter. Ainsi, si à cet instant on coupe l'alimentation du moteur, on observe un retour en arrière à une vitesse qui est grossièrement proportionnelle à l'énergie stockée, c'est-à-dire à la pression dans la poche.

De la sorte, si cette pression est faible, le retour du moteur est lent, la fréquence de la pompe est basse et le débit sanguin est faible. Inversement, une pression importante dans la poche conduit à un accroissement de la fréquence et du débit. La pompe connue réalise donc un asservissement hydraulique qui optimise son fonctionnement en fonction des conditions externes.

Un tel asservissement hydraulique de la pompe connue est également avantageux en ce que la consommation électrique moyenne du moteur est réduite, celui-ci étant dans certains cas alimenté seulement pendant la phase - la plus courte - de contraction systolique.

Pour assurer une maîtrise naturelle de ce phénomène à toutes les fréquences de fonctionnement, on choisit avantageusement une vitesse élevée de contraction pour que cette phase dure environ 200 ms, et une vitesse de retour plus lente, pour une durée d'environ 1000 ms, soit une fréquence basale d'environ 50 pulsations/minute.

Lorsque le retour veineux s'accroît, le remplissage de la poche augmente et la pression dans celle-ci également. On assiste donc à un accroissement de la vitesse de retour et à une augmentation de la fréquence de fonctionnement de la pompe pour assurer le débit.

Cependant cet asservissement hydraulique exige l'existence d'une pression aortique élevée, sans quoi la pression dans la poche est limitée à une valeur qui interdit un accroissement de la fréquence de fonctionnement au delà d'une certaine limite. Certes la pompe décrite dans la demande de brevet sus-visée permet, de par sa conception, un accroissement de fréquence en fonction du volume de remplissage; plus précisément, un accroissement de ce volume, pour une vitesse de contraction fixe, provoque un accroissement de la quantité de sang éjectée par unité de temps et de là un accroissement de la pression.

Mais l'expérience montre que cet effet complémentaire est insuffisant car sur un coeur naturel, la variation de débit en fonction du volume de remplissage est prépondérante.

Pour pallier cette difficulté, une solution connue consiste à utiliser un ou plusieurs capteurs de pression et/ou de débit dans la cavité auriculaire du coeur, et de commander la vitesse de retour pour accroître la fréquence de fonctionnement en cas de faible pression aortique. Cette solution est cependant lourde et dangereuse car il faut s'assurer de la bonne alimentation électrique des capteurs, ainsi que de leur fiabilité et de leur précision.

La présente invention vise à pallier ces inconvénients de la technique antérieure et à proposer un procédé et un dispositif de commande de moteur, dans une pompe cardiaque du type précité, qui ne nécessite pas l'implantation de capteurs spéciaux ni de modification de la construction physique de la pompe.

Elle concerne à cet effet un procédé de commande asservie d'un moteur de prothèse cardiaque péristaltique, ayant les particularités de la revendication 1.

L'invention propose également un dispositif ayant les particularités de la revendication 3.

D'autres aspects, buts et avantages de la présente invention apparaîtront mieux à la lecture de la description détaillée suivante d'une forme de réalisation préférée de celle-ci, donnée à titre d'exemple non limitatif et faite en référence aux dessins annexés, sur lesquels :
la figure 1 est une vue schématique en perspective d'un moteur de prothèse cardiaque piloté conformément à l'invention,
la figure 2 est la représentation électrique du moteur de la figure 1,
la figure 3 est un schéma-bloc du moteur et de sa commande,
la figure 4 représente une première partie d'un circuit de commande selon l'invention,
la figure 5 représente une seconde partie du circuit, et
les figures 6a, 6b, 7, 8a et 8b sont des diagrammes illustrant le fonctionnement d'une partie du circuit de l'invention.

On notera préliminairement que, d'une figure à l'autre, des éléments ou parties identiques ou similaires ont été désignés par les mêmes signes de référence.

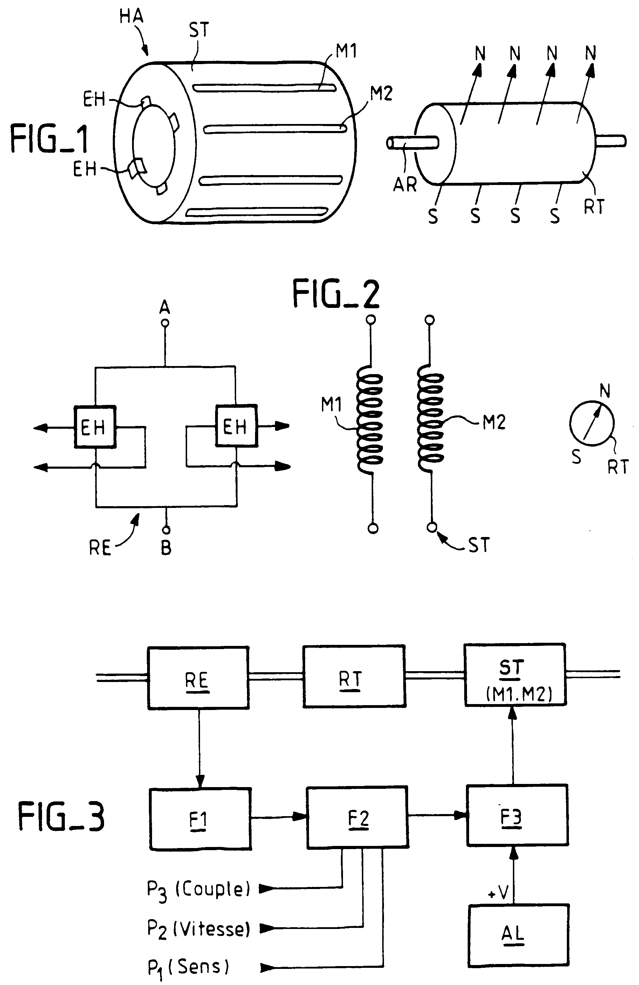

En référence tout d'abord aux figures 1 et 2, le moteur auquel s'applique le procédé de commande de la présente invention est un moteur sans collecteur, ni bague, ni balais, ceci afin d'atteindre le haut degré de fiabilité et la longue durée de vie nécessaires à l'application considérée. Il s'agit d'un moteur à commutations électroniques mono- ou multiphasé. Dans la description qui suit, on considèrera le cas d'un moteur biphasé, et tous les angles exprimés seront des angles électriques.

Le moteur comprend une partie tournante ou rotor RT, et une partie non tournante ou habitacle HA.

Le rotor RT comporte un ensemble d'aimants permanents (non illustrés) solidaires d'un arbre de rotation AR. Ils définissent un vecteur d'aimantation NS perpendiculaire à l'axe de rotation tout le long de l'arbre, comme illustré sur la figure 1.

L'arbre de rotation est utilisé comme arbre moteur et est maintenu sur un axe virtuel fixe et centré par rapport à l'habitacle HA au moyen par exemple de deux roulements à billes ou paliers (non illustrés) lui assurant une totale liberté de rotation. Pour plus de détails quant à l'implantation du moteur dans une pompe sanguine, on se réfèrera au contenu de la demande de brevet européen n° 0 148 661 au nom des Demandeurs.

L'habitacle HA comprend deux sous-ensembles fixés l'un à l'autre et présentant un axe de symétrie complet par rapport à l'axe de rotation du rotor.

Un premier sous-ensemble constitue un stator ST constitué d'un ensemble d'enroulements de fil de cuivre conçus de manière à être équivalents à un couple de bobines M1 et M2 capables de produire un champ magnétique tournant en fonction de la polarité du courant circulant dans l'une et/ou l'autre de ces bobines, ce champ tournant étant toujours perpendiculaire à l'axe de rotation du rotor.

Le moteur ne comportant pas de collecteur, on prévoit des moyens spécifiques pour connaître à tout instant la position angulaire du rotor, c'est-à-dire l'orientation de son vecteur de champ magnétique, par rapport à l'habitacle. A cet effet, le second sous-ensemble de l'habitacle consiste en un résolveur RE. Il est constitué de bobines ou de cellules à effet Hall EH, en l'espèce au nombre de deux.

On va maintenant décrire en référence à la figure 3 le principe de commande électronique du moteur.

Dans un premier bloc F1, on mesure, à partir des signaux délivrés par les cellules du résolveur RE, la position angulaire du rotor. Le bloc F2 a pour fonction d'élaborer la configuration du courant électrique à faire circuler dans les bobines M1 et M2 pour obtenir à la fois le couple moteur souhaité, le sens de rotation souhaité et la vitesse de rotation souhaitée, ces paramètres étant fournis sous forme de valeurs de consigne appliquées à trois entrées spécifiques du bloc F2.

Le bloc F3 assure la distribution des courants électriques aux bobines M1 et M2 du stator. Il comporte à cet effet un circuit de puissance commandé par le bloc F2 et recevant son alimentation depuis un boîtier d'alimentation en courant continu approprié, noté AL.

Le bloc F2 détermine les courants à faire circuler dans le stator à partir de trois paramètres P1, P2, P3. P1 est un signal logique déterminant le sens de rotation du moteur, avant ou arrière. P2 est un signal dont la valeur est représentative de la vitesse maximale autorisée en rotation avant, ainsi que du couple maximal associé, fonction également de la puissance électrique fournie par le bloc F3. P3 est un signal dont la valeur est représentative de la vitesse de consigne en rotation arrière.

Le couple moteur fourni sur l'arbre AR est une fonction directe de la puissance électrique fournie au bloc F3 par le boîtier d'alimentation AL. En intégrant dans les calculs le rendement du moteur, de la mécanique associée de la pompe et le rendement électrique du bloc de puissance F3, on peut donc obtenir une relation qui exprime le couple fourni en fonction du courant consommé par le bloc F3.

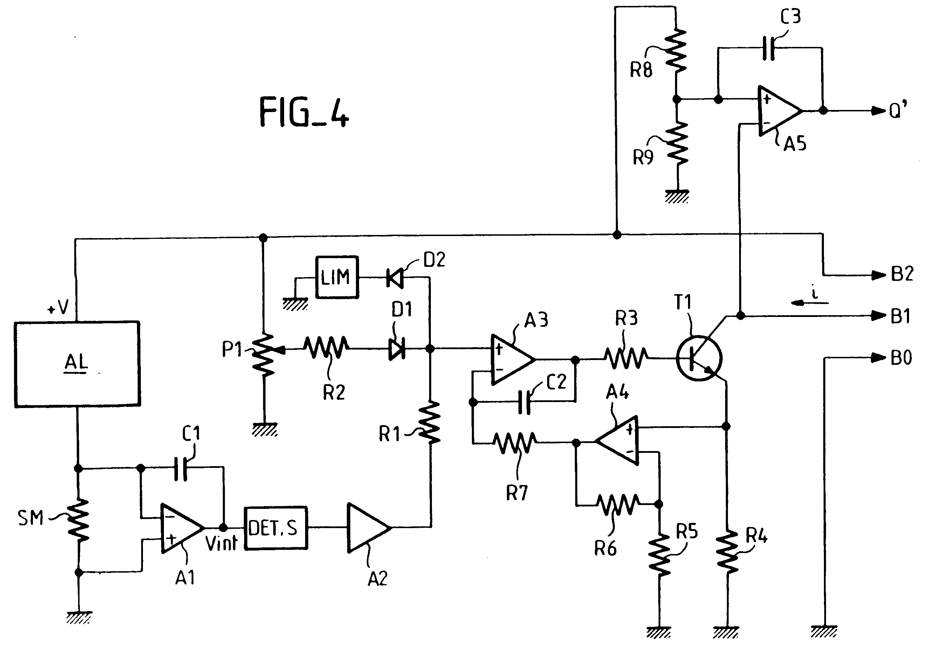

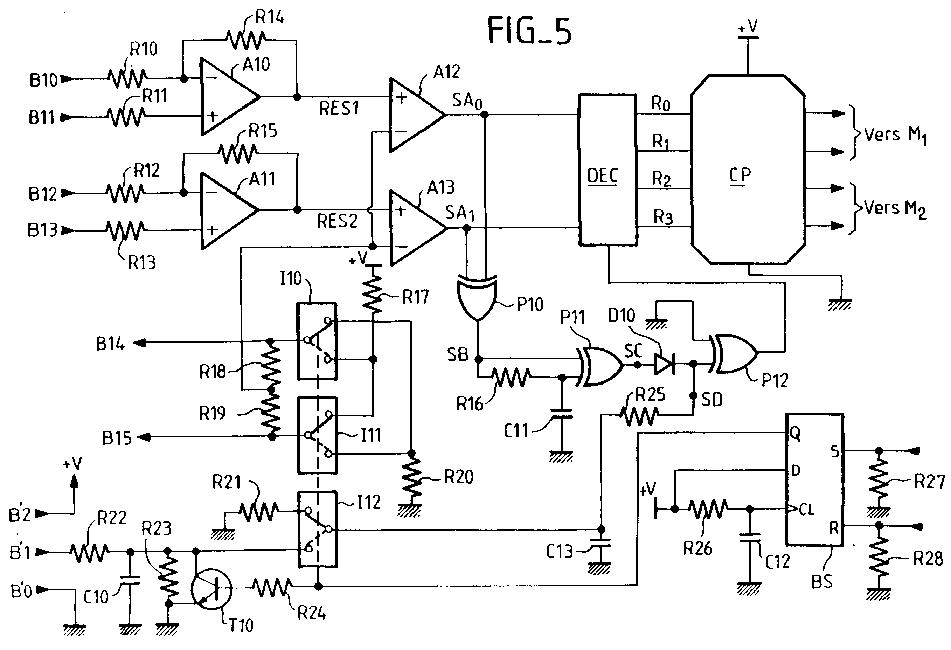

On va maintenant décrire en référence aux figures 4 et 5 un exemple concret de circuit électrique réalisant les fonctions des blocs F1, F2 et F3.

Le bloc d'alimentation électrique AL est constitué par exemple par une ou plusieurs piles de capacité appropriée, et fournit par exemple une tension continue de +12 volts, notée +V.

Une résistance de faible valeur formant shunt de mesure SM est interposée entre la borne négative du bloc AL et la masse.

Les deux bornes de SM sont respectivement appliquées aux entrées non-inverseuse et inverseuse d'un amplificateur opérationnel A1 monté en amplificateur/intégrateur, comportant un condensateur C1 entre son entrée inverseuse et sa sortie.

Le bloc DET.S combine un intégrateur à longue constante de temps (par exemple de l'ordre de 10 secondes) avec un détecteur à seuil situé en amont de l'intégrateur, à des fins expliquées plus loin.

La signal de sortie de DET.S est amplifié par l'amplificateur opérationnel A2 dont le gain détermine, comme on le verra plus loin, la pente de la caractéristique de contrôle automatique. La sortie de A2 est reliée à l'entrée non-inverseuse d'un autre amplificateur opérationnel A3 via une résistance R1. Un potentiomètre de réglage manuel P1 a son curseur relié via une résistance R2 et une diode D1 à l'entrée non-inverseuse de A3. Enfin un circuit limiteur LIM est également relié via une diode D2 à ladite entrée.

L'amplificateur opérationnel A3 est monté en amplificateur intégrateur à gain élevé, un condensateur C2 étant monté entre sa sortie et son entrée inverseuse. Sa sortie est reliée à la base d'un transistor bipolaire NPN T1 via une résistance R3. L'émetteur de T1 est relié à la masse via une résistance d'émetteur R4 et est également relié à l'entrée non-inverseuse d'un amplificateur opérationnel A4. L'entrée inverseuse de A4 est reliée à la masse via une résistance R5 et à sa sortie via une résistance R6. La sortie de A4 est par ailleurs reliée via une résistance R7 à l'entrée inverseuse de A3.

Le collecteur de T1 est relié à une borne de sortie B1 du circuit, ainsi qu'à l'entrée inverseuse d'un amplificateur opérationnel A5. L'entrée non-inverseuse de A5 reçoit une fraction déterminée de la tension +V par l'intermédiaire d'un pont diviseur, tandis qu'un condensateur C3 est relié entre ladite entrée non-inverseuse et la sortie de A5, cette dernière étant reliée à une borne de sortie Q′. Cette sortie constitue, comme on le verra plus loin, une indication de la position de l'étrier de la pompe cardiaque.

Le circuit comporte deux autres bornes de sortie B2 et B0, reliées respectivement à +V et à la masse.

Les bornes de sortie B0 à B2 sont destinées à la connexion, via un câblage approprié, à la partie du circuit représentée sur la figure 5 et intégrée à la prothèse.

La borne B1 constitue le "troisième fil" par lequel est véhiculé un courant noté i sur la figure 4, qui constitue un signal de consigne de vitesse de retour élaboré à partir de la valeur moyenne du courant dans le moteur.

On va maintenant décrire en référence à la figure 5 la partie de circuit située dans la prothèse. Les bornes B′0, B′1 et B′2 sont respectivement reliées par des conducteurs souples appropriés aux bornes B0, B1, B2 de la figure 4.

Des bornes B10, B11, B12 et B13 reçoivent les signaux de sortie des deux sondes à effet Hall EH du moteur. Ces signaux sont appliqués, via des résistances R10, R11, R12 et R13 aux bornes d'entrée de deux amplificateurs opérationnels A10 et A11, respectivement associés aux deux sondes. Ces amplificateurs sont montés en différentiateurs, avec deux résistances de rétroaction R14 et R15 montés entre leurs sorties et leurs entrées inverseuses. Les sorties de A10 et A11 sont respectivement. reliées aux entrées non-inverseuses de deux comparateurs A12 et A13, dont les entrées inverseuses sont reliés à un circuit générateur de tension médiane décrit plus loin. Les sorties de A12 et A13 sont respectivement reliées aux deux entrées d'un décodeur à quatre voies DEC, ainsi qu'aux deux entrées d'une première porte OU-EXCLUSIF P10. La sortie de P10 est appliquée directement à une première entrée d'une seconde porte OU-EXCLUSIF P11, et à la seconde entrée de cette porte via une résistance R16. Un condensateur C11 est prévu entre cette seconde entrée et la masse. La sortie de P11 est reliée via une diode D10 montée en direct à une première entrée d'une troisième porte OU-EXCLUSIF P12 dont l'autre entrée est reliée à la masse. Ladite première entrée de P12 est connectée à la masse via une résistance R25 et un condensateur C13. La sortie de P12 est reliée à l'entrée de commande du décodeur DEC. Les quatre sorties parallèles de DEC sont appliquées à un circuit de puissance PC, par exemple un circuit hybride de type conventionnel, chargé d'appliquer aux enroulements M1 et M2, selon le cadencement défini par l'état de ses entrées, les intensités appropriées dans des sens appropriés.

Le circuit d'alimentation des sondes à effet Hall et de génération de la tension médiane précitée est construit autour de deux inverseurs commandés, par exemple des inverseurs de type C-MOS, respectivement I10 et I11, qui sont capables d'engendrer entre deux bornes de sortie B14 et B15 une tension alternativement positive et négative. Deux résistances R18 et R19 montées en série entre ces deux bornes permettent d'appliquer aux entrées inverseuses de A12 et A13 la moitié de cette tension, formant le seuil de la comparaison.

La borne B′1 est reliée à un premier contact fixe d'un autre inverseur commandé I12 via une résistance R22. L'autre contact fixe est relié à la masse via une résistance R21. Le contact mobile de I12 est relié au point commun entre R25 et C13. Une résistance R23 et un condensateur C10 sont montés entre la masse et le premier contact fixe de I12. Un transistor bipolaire de type NPN T10 a son collecteur relié également à ce premier contact fixe, tandis que son émetteur est relié à la masse et sa base est reliée via une résistance R24 à la sortie Q d'une bascule de type D à front montant, notée BS, cette sortie commandant également le basculement simultané des trois inverseurs I10, I11 et I12.

Les entrées d'armement (S) et de réarmement (R) de la bascule BS sont reliées à des bornes d'entrée B16 et B17 pour des signaux fournis par deux capteurs de fin de course de la pompe cardiaque, ces capteurs étant décrits dans la demande de brevet européen précitée.

On va maintenant expliquer le fonctionnement du dispositif de la présente invention.

Supposons tout d'abord que la pompe est dans sa phase de retour. Selon un aspect essentiel de la présente invention, la vitesse de retour doit se calquer sur une vitesse de consigne elle-même fixée en fonction de l'énergie consommée par le moteur pendant les phases d'éjection.

La valeur de cette énergie est obtenue par intégration du courant i consommé par le moteur. Plus précisément, la résistance SM présente entre ses bornes une tension proportionnelle au courant. Cette tension est amplifiée par A1, qui par son rôle d'intégrateur élimine en outre les transitoires non significatives, et à la sortie duquel est produite une tension instantanée Vint proportionnelle au courant.

Le circuit DET.S est un détecteur/intégrateur à constante de temps longue, par exemple de l'ordre de 10 secondes. Le détecteur (circuit à seuil) a pour objet d'éviter de prendre en compte dans l'intégration des courants résiduels ou non significatifs (en particulier le courant consommé lors de les mouvements de retour de la pompe (rotation arrière). L'intégrateur permet de produire à la sortie de DET.S une tension qui est représentative de l'effort moyen sur un certain nombre de pulsations cardiaques. Plus précisément, la vitesse de contraction systolique étant choisie constante, comme on l'explicitera plus loin, le couple développé par le moteur pendant ces phases est proportionnel à la quantité de sang éjectée par unité de temps, c'est-à-dire au volume de remplissage. On obtient donc de cette manière une indication du débit souhaité par la physiologie.

Le gain de A2, qui peut par exemple être modifié à l'aide d'une résistance variable appropriée (non illustrée), permet de déterminer la pente du contrôle automatique en modifiant le gain de la boucle de réaction.

A3 additionne et intègre les tensions issues d'une part de A2, et d'autre part de P1, ce potentiomètre constituant un élément de réglage manuel. Le circuit LIM en coopération avec la diode D2 permet de fixer pour la tension appliquée à A3 une limite supérieure à ne pas dépasser, à des fins expliquées plus loin.

La sortie de A3 polarise la base de T1, et permet donc de contrôler le courant i capable de circuler dans ce transistor depuis la borne de troisième fil B1, via la résistance R4 et vers la masse.

Ainsi le circuit de la figure 4 a pour fonction essentielle d'autoriser la circulation à travers T1 d'un courant qui croît, selon une courbe spécifique (approximativement une droite), avec le couple produit par le moteur. La pente générale de cette courbe est fixée par le gain de A2, tandis que la hauteur de cette courbe est fixée par le potentiomètre P1. Enfin le circuit limiteur permet d'éviter que la zone supérieure de cette courbe excèdent un plafond déterminé, en la "forçant" à ce plafond.

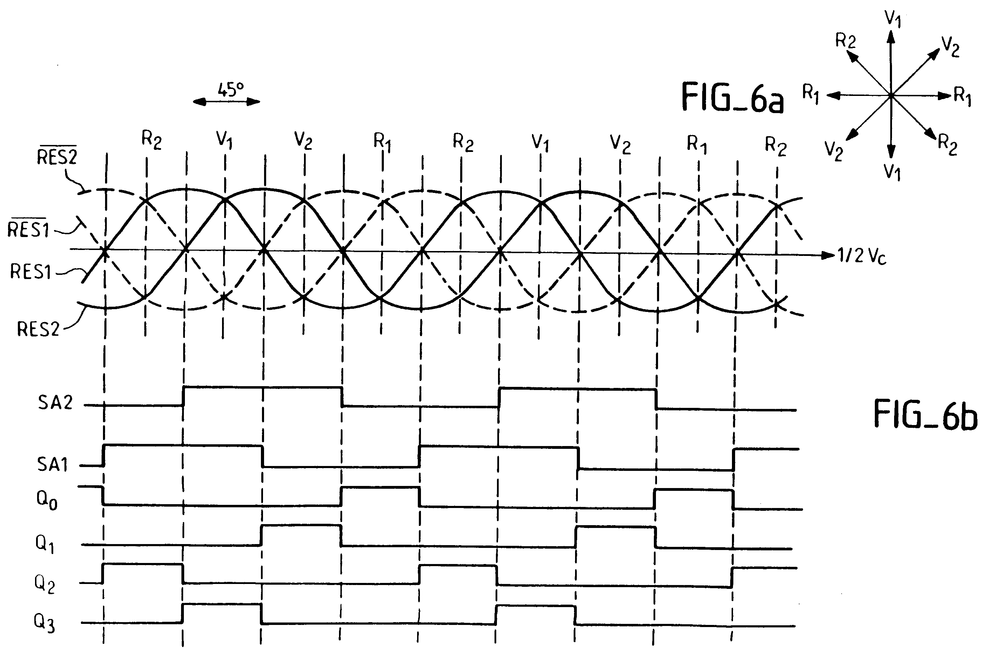

On va maintenant décrire le fonctionnement de la partie de circuit représentée sur la figure 5, en référence aux chronogrammes des figures 6b, 7, 8a et 8b. La figure 6a illustre la répartition angulaire des positions d'équilibre du rotor par rapport aux deux enroulements M1, M2 alimentés pour la marche avant (respectivement V1 et V2) et pour la marche arrière (respectivement R1 et R2). On outre, sur les chronogrammes, le sens de gauche à droite correspond à la marche avant du moteur (phase d'éjection), tandis que le sens de droite à gauche correspond à la marche arrière (phase de retour).

Les inverseurs commandés I10 et I11 permettent d'appliquer aux cellules à effet Hall des tensions de polarisation alternativement dans un sens et dans l'autre. La tension recueillie au point commun entre R18 et R19, toutes deux de même valeur, constitue la tension médiane Vc de cette polarisation.

Les amplificateurs différentiels A10 et A11 délivrent sur leurs sorties les tensions produites par les capteurs à effet Hall, en forme de sinusoïdes, représentées en traits continus et désignées par RES1 et RES2 sur les figures 5 et 6a. Les tensions inverses RES1 et RES2 sont représentées en tiretés.

Les amplificateurs A12 et A13 sont montés en comparateurs, leur seuil commun étant la tension médiane précitée. Ils délivrent donc sur leurs sorties respectives des signaux carrés, notés SA1 et SA2, qui sont à un niveau haut (tension de + 12 volts) pendant les demi-ondes positives des signaux RES1 et RES2 et à un niveau logique bas (tension nulle) pendant les demi-ondes négatives.

Ces signaux sont appliqués aux deux ent.rées de signaux du décodeur DEC, ainsi qu'aux deux entrées de la porte P10.

Le décodeur DEC a pour objet, par une logique combinatoire appropriée, d'engendrer des signaux logiques de position Q0, Q1, Q2, Q3 (figure 6b) qui sont respectivement associés aux quatre positions d'équilibre du moteur.

On observe ici, selon un autre aspect intéressant de la présente invention, que la position des capteurs EH est choisie de manière que les indications de positions puissent être dérivées de signaux liés seulement aux signes des signaux produits par les capteurs. Ceci est obtenu par un positionnement des capteurs EH avec un décalage, par rapport aux bobines M1 et M2, tel que les capteurs (et donc les extréma des signaux qu'ils produisent) soient angulairement à mi-chemin entre deux positions d'équilibre du rotor.

Dans le cas d'espèce d'un moteur biphasé, la valeur de ce décalage est de 22°5.

Conventionnellement, de tels capteurs étaient positionnés en superposition avec les enroulements et en corollaire, les fronts des signaux logiques de position engendrés devaient être dérivés des crêtes, relativement plateau des signaux à peu près sinusoïdaux délivrés par les capteurs. La précision obtenue était médiocre.

Grâce à cet aspect de l'invention, les fronts des signaux de position sont obtenus avec une très bonne précision et, de façon particulièrement appropriée pour la commande du moteur telle qu'on va la décrire ci-dessous, sont situés en des positions angulaires exactement médianes entre les positions d'équilibre du moteur. Plus précisément, chaque signal Q0 à Q3 passe au niveau haut à une position médiane entre une position d'équilibre voisine et la position d'équilibre associée, et repasse au niveau bas à une position médiane entre la position d'équilibre associée et la position d'équilibre suivante.

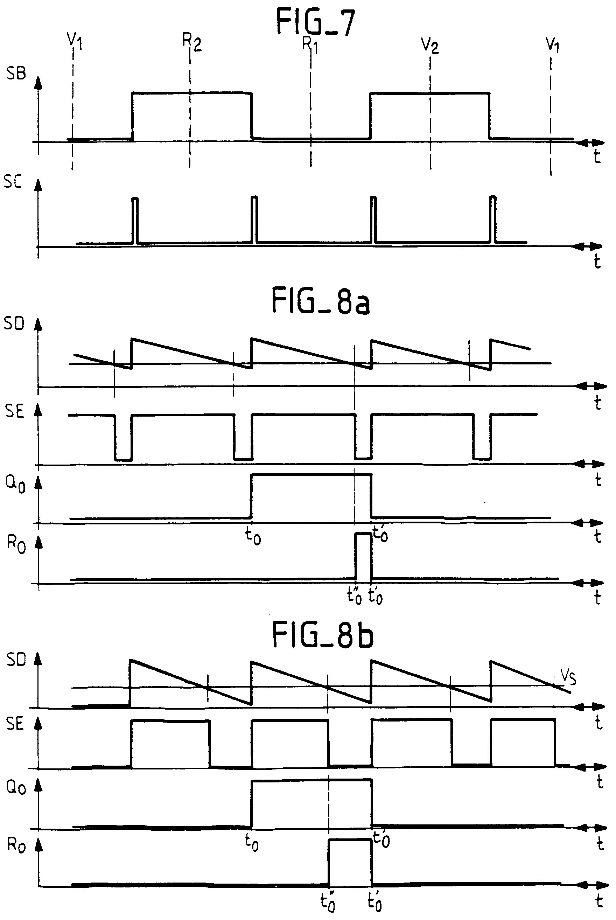

La porte P10 délivre sur sa sortie un signal rectangulaire SB tel qu'illustré sur la figure 7. La porte P11 a pour objet de produire à sa sortie une impulsion, de faible largeur déterminée par les valeurs de R16 et de C11, à chaque front montant et à chaque front descendant de SB (signal SC sur la figure 7). Ainsi on trouve une telle impulsion à chaque position angulaire intermédiaire entre deux positions d'équilibre du moteur (ces positions étant notées V1, V2 pour la marche en avant et R1, R2 pour la marche en arrière), les impulsions étant séparées par des intervalles angulaires (électriques) de 45° et en avance de 22,5° par rapport à chaque position d'équilibre.

On va supposer maintenant que le moteur est en phase de retour. Selon un aspect essentiel de l'invention, la vitesse de retour est déterminée par l'effort cardiaque à fournir, cet effort étant représenté comme on l'a expliqué plus haut par la valeur du courant i qui peut circuler dans le transistor T1. Dans cette situation, la bascule BS délivre un signal logique Q de niveau bas et les interrupteurs commandés I10, I11 et I11 sont dans la position illustrée en tiretés sur la figure 5. Le transistor T10 est bloqué.

La figure 8a illustre le cas où l'effort moyen fourni par le moteur durant les cycles précédents était faible, de telle sorte que le courant qui peut circuler dans T1 est également faible. Dans ce cas, le signal SD à la liaison entre D10 et R25, appliqué à la seconde entrée de la porte P12, prend l'allure d'une tension en dents de scie dont la pente de décroissance est faible. Plus précisément, lorsqu'une impulsion du signal SC se présente à la sortie de P11, le condensateur C13 se charge rapidement via la résistance R25 de valeur relativement faible. A la disparition de l'impulsion, C13 va se décharger par le seul circuit possible, c'est-à-dire via I12, R22, T1 et R4, et donc à une vitesse déterminée par le courant autorisé dans T1 et en l'espèce lentement.

L'autre borne de P12 étant à la masse, la sortie de P12 restitue un signal logique SE dont le rapport cyclique varie en fonction de la vitesse de décroissance du signal SD. Dans le cas d'espèce, on observe de larges impulsions de niveau logique haut, correspondant aux instants pendant lesquels la tension SD est supérieure à la tension de seuil Vs pour laquelle la sortie de P12 bascule d'un niveau à l'autre.

La tension SE est appliquée à une entrée de commande du circuit DEC, où elle est combinée selon une fonction logique appropriée avec chaque signal Q0 à Q3, de manière à retarder le front montant du signal Qi considéré jusqu'à l'instant où se produit le front descendant de SE. La figure 8a illustre le signal Q0, avec ses instants t0 et t0′ de front montant et de front descendant. Le signal R0 engendré à partir de Q0 et de SE est une impulsion plus étroite dont le front montant est à l'instant t0˝ correspondant au front descendant de SE et dont le front descendant reste à l'instant t0′.

Dans le cas de la figure 8b, l'effort moyen fourni par le moteur au cours des cycles précédents était important. Le courant i qui peut circuler dans T1 est donc important, et la vitesse de décroissance du signal SD est rapide. Les impulsions de SE sont donc plus brèves, si bien que les impulsions des signaux Ri (en l'espèce le signal R0) débutent beaucoup plus tôt que dans le cas de la figure 8a, et sont donc beaucoup plus larges.

Ainsi, dans le cas de la figure 8b, le courant moyen fourni au moteur est important, du fait du rapport cyclique élevé des signaux Ri appliqués au circuit de puissance CP.

Lorsque, à partir de la situation de la figure 8b, l'effort moyen fourni diminue, c'est-à-dire que le courant consommé par le moteur pendant les phases d'éjection diminue, la tension à l'entrée de A3 diminue et le courant i également. La situation tend donc à évoluer vers celle de la figure 8a, pour ainsi ralentir la vitesse de retour et diminuer la fréquence de fonctionnement de la pompe.

On peut observer ici que, en particulier dans les phases d'effort faible, du courant est fourni au moteur pendant des périodes correspondant à des positions intermédiaires entre les positions d'équilibre successives. De cette manière, on donne au moteur un bon rendement en travaillant aussi loin que possible des positions d'équilibre.

Un avantage essentiel du circuit décrit réside en ce qu'il effectue une auto-régulation de la vitesse effective du moteur sur la vitesse de consigne. Plus précisément, considérons le cas où le moteur fonctionne selon le régime de la figure 8a et que la vitesse de rotation du moteur commence à croître. On suppose également que l'effort moyen fourni par le moteur n'a pas changé et donc que la vitesse de décroissance du signal SD reste la même.

Dans ce cas, la durée de chaque cycle du signal SE diminue, mais la durée de la phase de niveau haut de ce cycle reste la même puisque la pente du signal SD reste la même. Le rapport cyclique de SE augmente donc, et il en résulte que le rapport cyclique de R0 (et des autres signaux R1-R3) diminue, pour abaisser la quantité de courant fournie par le moteur, et donc le couple fourni et donc, pour des conditions extérieures identiques, la vitesse.

Le phénomène de régulation inverse se produit lorsque la vitesse effective du moteur diminue par rapport à la vitesse de consigne déterminée par le courant i.

On va maintenant décrire le fonctionnement du circuit des figures 4 et 5 en phase d'éjection systolique. Le début de cette phase est déclenché par l'action du détecteur de fin de course arrière, qui a appliqué à la bascule BS une impulsion d'armement. La sortie Q de BS est au niveau haut, par exemple +12 volts.

Les inverseurs commandés I10, I11, I12 passent dans les positions indiquées en traits pleins. Du fait de l'inversion des signaux appliqués aux résolveurs, l'ordonnancement temporel des signaux Q0 à Q3 est inversé, pour faire fonctionner le moteur dans le sens avant. Les portes P10 à P12 jouent le même rôle que précédemment, à ceci près que C13 se décharge non plus via T1 mais à une vitesse constante fixée par la valeur de la résistance R21. La pente de décroissance des signaux SD est donc invariable, tout comme le rapport cyclique des signaux SE et donc des signaux R0 à R3 appliqués au circuit de puissance. Par exemple, ces signaux peuvent débuter dans la position médiane entre la position d'équilibre précédente et la position d'équilibre associée (comme dans le cas de la marche en arrière) et se terminer au voisinage de la position d'équilibre associée.

On réalise de cette manière une phase d'éjection à vitesse constante, occupant par exemple une durée de l'ordre de 200 ms.

Pendant la phase d'éjection, le circuit de l'invention réalise une autre fonction, selon laquelle les informations de position délivrées par les deux détecteurs de fin de course de la pompe, qui sont transcrites au niveau de la sortie Q de la bascule BS, sont ramenées dans le circuit de la figure 4.

Plus précisément, pendant la phase de retour décrite plus haut, un certain courant circule dans T1 et son collecteur est à une certaine tension positive. Les valeurs de R8 et R9 sont choisies pour que, quelle que soit la valeur de i, la tension au collecteur de T1, appliquée à l'entrée inverseuse de A5, soit toujours supérieure à la tension appliquée sur l'entrée non-inverseuse de A5. Ce dernier délivre donc sur sa sortie Q' un signal logique de niveau bas, signalant que le moteur est en marche arrière.

Inversement, pendant la phase d'éjection, le courant de décharge de C13 ne circule plus dans T1, et les bornes B1 et B'1 sont amenées à une tension proche de zéro plus du fait que T10 est alors rendu passant. Cette tension est inférieure à celle de l'entrée non-inverseuse de A5, si bien que ce dernier délivre maintenant sur Q' un niveau logique haut.

Ainsi chaque passage de Q' au niveau haut indique le début d'une phase d'éjection. Cette information, délivrée sur une borne de connexion appropriée externe au porteur, permet donc de mesurer facilement la fréquence cardiaque et le débit de la prothèse.

## Revendications

1. Procédé de commande asservie d'un moteur de prothèse cardiaque péristaltique à commutations électroniques,
caractérisé en ce qu'il comprend les étapes suivantes :
- pendant une phase d'éjection, appliquer au moteur un courant provoquant sa rotation dans un premier sens à vitesse essentiellement constante, le couple développé par le moteur étant proportionnel à la quantité de sang éjectée par unité de temps, et déterminer le couple développé par le moteur, pour obtenir une information relative au débit sanguin demandé par le corps humain, en mesurant le courant moyen consommé par le moteur, et
- pendant une phase de retour, appliquer au moteur un courant variable, en jouant sur la largeur d'impulsions de courant appliquées au moteur, pour provoquer sa rotation dans le sens opposé à une vitesse d'autant plus élevée que le débit sanguin demandé, représenté par le courant moyen mesuré, est élevé.

2. Procédé selon la revendication 1, caractérisé en ce que le courant moyen est établi sur une pluralité de cycles de fonctionnement du moteur.

3. Dispositif de commande asservie d'un moteur de prothèse cardiaque péristaltique à commutations électroniques, caractérisé en ce qu'il comprend des moyens (F1, F2, F3) pour appliquer au moteur :
- pendant une phase d'éjection, un courant provoquant sa rotation dans un premier sens à vitesse essentiellement constante, et
- pendant une phase de retour, un courant provoquant sa rotation dans le sens opposé à une vitesse d'autant plus élevée que le courant moyen circulant dans le moteur, représentatif de l'effort moyen fourni par le moteur, est élevé,
et en ce qu'il comprend en outre :
- des premiers moyens capteurs (SM, A1-A4) pour engendrer un signal électrique proportionnel au courant moyen circulant dans le moteur,
- des seconds moyens capteurs (EH, A12, A13) pour engendrer des signaux de position (Q0-Q3) représentatifs de la position angulaire d'un rotor (RT) du moteur,
- des moyens de commande de largeur de signal (P10-P12, C13) capables
pendant une phase de retour, de produire des signaux de commande (SE) dont la largeur varie en fonction dudit signal électrique proportionnel au courant moyen, et
pendant une phase d'éjection, de produire des signaux de commande dont la largeur est fixe,
- un circuit logique (DEC) combinant les signaux indicateurs de position et les signaux de commande pour produire des signaux de commutation de courant (R0-R3),
- un circuit de puissance (CP) recevant lesdits signaux de commutation de courant et appliquant à au moins un enroulement du moteur un courant dont la répartition temporelle dépend desdits signaux de commutation et détermine la vitesse de rotation du moteur.

4. Dispositif selon la revendication 3, caractérisé en ce que les premiers moyens capteurs comprennent:
une résistance (SM) placée en série sur le trajet de courant vers le moteur, et
des moyens d'amplification et d'intégration (A1-A3, DET.S) recevant la tension aux bornes de la résistance.

5. Dispositif selon la revendication 4, caractérisé en ce que les moyens d'amplification et d'intégration comprennent un intégrateur (DET.S) dont la constante de temps est égale à la durée d'une pluralité de cycles de fonctionnement de la pompe.

6. Dispositif selon la revendication 5, caractérisé en ce que l'intégrateur (DET.S) est associé à un détecteur à seuil.

7. Dispositif selon la revendication 4, 5 ou 6, caractérisé en ce que les premiers moyens capteurs comprennent en outre des moyens de réglage manuel (P1) pour faire varier la pente et/ou la hauteur d'une courbe monotone liant ledit signal électrique au courant moyen.

8. Dispositif selon la revendication 7, caractérisé en ce que les premiers moyens capteurs comprennent en outre des moyens limiteurs (LIM) pour plafonner ladite courbe monotone.

9. Dispositif selon l'une des revendications 4 à 8, caractérisé en ce que les premiers moyens capteurs sont conçus pour faire varier la valeur d'un courant électrique autorisé à circuler dans un trajet de courant (T1, R4).

10. Dispositif selon la revendication 9, caractérisé en ce que les moyens de commande de largeur de signal comportent un condensateur (C13) capable, pendant les phases de retour, d'être chargé ou déchargé par un courant (i) circulant dans ledit trajet, et un moyen à seuil (P12) revecant la tension aux bornes du condensateur.

11. Dispositif selon la revendication 10, caractérisé en ce qu'il est prévu des moyens de commutation (I12) capables, en fonction du sens de rotation du moteur, de relier le condensateur (C13) alternativement audit trajet de courant et à une résistance de valeur fixe (R21).

12. Dispositif selon la revendication 11, caractérisé en ce que les moyens de commutation sont commandés par un circuit (BS) relié à des capteurs de fin de course d'un élément mobile de la pompe.

13. Dispositif selon l'une des revendications 3 à 12, pour un moteur sans collecteur, caractérisé en ce que les seconds moyens capteurs comprennent au moins un capteur magnétique (EH) monté sur un stator du moteur et des moyens comparateurs (A12, A13) pour engendrer des signaux logiques de position (SA1, SA2) variant avec le signe des signaux fournis par le ou les capteurs magnétiques.

14. Dispositif selon la revendication 13, elle-même rattachée à la revendication 11, caractérisé en ce que des moyens de commutation (I10, I11) sont également reliés aux seconds moyens capteurs pour modifier le déroulement temporel des signaux de position (Q0-Q3) avec l'inversion du sens de rotation du moteur.

15. Dispositif selon la revendication 13 ou 14, caractérisé en ce que le ou les capteurs magnétiques (EH) sont positionnés sur le stator de manière que des fronts délimitant les signaux de position soient situées dans des positions angulaires médianes entre des positions d'équilibre (V1, V2, R1, R2) du moteur

16. Dispositif selon la revendication 15, pour un moteur biphasé, caractérisé en ce que les capteurs magnétiques (EH) sont positionnés sur le stator avec un décalage angulaire de 22°5 par rapport aux positions d'équilibre du moteur.

17. Dispositif l'une des revendications 13 à 16, caractérisé en ce que les capteurs magnétiques sont des cellules à effet Hall (EH).

18. Dispositif selon l'une des revendications 3 à 17, caractérisé en que le circuit logique (DEC) engendre des signaux de commutation de courant (R0-R3) provoquant l'application de courant à l'enroulement ou aux enroulements du moteur à partir ou jusqu'à un instant (t0') correspondant à une position angulaire médiane entre deux positions d'équilibre du moteur.

19. Dispositif selon l'une des revendications 3 à 18, comprenant une première partie, extérieure, abritant les premiers moyens capteurs et une seconde partie, implantée, abritant les seconds moyens capteurs, les moyens de commande de largeur de signal, le circuit logique et le circuit de puissance, et des câbles souples de liaison entre les deux parties comprenant une liaison (B1, B'1) entre les premiers moyens capteurs et les moyens de commande de largeur de signal.

20. Dispositif selon la revendication 19, caractérisé en ce que, pendant les phases d'éjection, ladite liaison (B1, B'1) est utilisée pour véhiculer vers la première partie du dispositif une information de début de phase d'éjection engendrée dans la seconde partie du dispositif.

21. Dispositif selon la revendication 20, elle-même rattachée à la revendication 12, caractérisé en ce que la seconde partie du dispositif comprend un moyen de commutation (T10) commandé par la sortie du circuit (BS) relié aux capteurs de fin de course pour amener ladite liaison à une tension déterminée pendant les phases d'éjection, et en ce que la première partie du dispositif comprend un moyen comparateur (A5) recevant la tension sur ladite liaison.

## Claims

1. A method of servo-controlling an electronically-switched motor of a peristaltic heart prosthesis, the method being characterized in that it comprises the following steps:
during an ejection stage, applying current to the motor that causes the motor to rotate in a first direction at a speed that is essentially constant, the torque developed by the motor being proportional to the quantity of blood ejected per unit of time, and determining the torque developed by the motor, in order to obtain information relating to the flow of blood required by the human body by measuring the average current consumed by the motor; and
applying a variable current to the motor during a return stage by acting on the width of current pulses applied to the motor, in order to cause it to rotate in the opposite direction at a speed which increases with increasing blood demand, as represented by the measured average current.

2. A method according to claim 1, characterized in that the mean current is established over a plurality of operating cycles of the motor.

3. Apparatus for servo-controlling the electronically-switched motor of a peristaltic heart prosthesis, the apparatus being characterized in that it comprises means (F1, F2, F3) for applying to the motor:
current that causes it to rotate in a first direction at a substantially constant speed during an ejection stage; and
current that causes it to rotate in the opposite direction during a return stage at a speed which increases with increasing mean current flow through the motor, which current flow is representative of the mean force delivered by the motor;
and in that it also comprises:
first sensor means (SM, A1-A4) for generating an electrical signal proportional to the mean current flowing through the motor;
second sensor means (EH, A12, A13) for generating position signals (Q0-Q3) representative of the angular position of a rotor (RT) of the motor;
signal width control means (P10-P12, C13) capable:
during a return stage, of producing control signals (SE) of width varying as a function of said electrical signal proportional to the mean current; and
during an ejection stage, of producing control signals of fixed width;
a logic circuit (DEC) combining the position-indicating signals and the control signals to produce current switching signals (R0-R3); and
a power circuit (CP) receiving said current switching signals and applying current to at least one winding of the motor with time sequencing that depends on said switching signals and that determines the speed of rotation of the motor.

4. Apparatus according to claim 3, characterized in that the first sensor means comprise:
a resistor (SM) connected in series in the path of current to the motor; and
amplification and integration means (A1-A3, DET.S) receiving the voltage across the terminals of the resistor.

5. Apparatus according to claim 4, characterized in that the amplification and integration means comprise an integrator (DET.S) whose time constant is equal to the duration of a plurality of operating cycles of the pump.

6. Apparatus according to claim 5, characterized in that the integrator (DET.S) is associated with a threshold detector.

7. Apparatus according to claim 4, 5, or 6, characterized in that the first sensor means further include manual adjustment means (P1) for varying the slope and/or the height of a monotonic curve relating said electrical signal to said mean current.

8. Apparatus according to claim 7, characterized in that the first sensors further include limiter means (LIM) for applying a ceiling to said monotonic curve.

9. Apparatus according to any one of claims 4 to 8, characterized in that the first sensor means are designed to vary the value of an electric current that is allowed to flow along the current pass (T1, R4).

10. Apparatus according to claim 9, characterized in that the means for controlling the width of the signal include a capacitor (C13) capable during the return stages of being charged or discharged by a current (i) flowing along said path, and threshold means (P12) receiving the voltage across the terminals of the capacitor.

11. Apparatus according to claim 10, characterized in that switching means (I12) are provided that are capable, as a function of the direction of rotation of the motor, of connecting the capacitor (C13) alternately to said current path and to a fixed value resistance (R21).

12. Apparatus according to claim 11, characterized in that the switching means are controlled by a circuit (BS) connected to end-of-stroke sensors for the moving elements of the pump.

13. Apparatus according to any one of claims 3 to 12, for a motor without a commutator, the apparatus being characterized in that the second sensor means comprise at least one magnetic sensor (EH) mounted on a stator of the motor and comparator means (A12, A13) for generating logic position signals (SA1, SA2) that vary with the sign of the signals provided by the magnetic sensor(s).

14. Apparatus according to claim 13, as dependent on claim 11, the apparatus being characterized in that the switching means (I10, I11) are also connected to second sensor means for modifying the time sequencing of the position signals (Q0-Q3) when the direction of rotation of the motor reverses.

15. Apparatus according to claim 13 or 14, characterized in that the magnetic sensor(s) (EH) is/are positioned on the stator in such a manner that the edges that delimit the position signals are situated at mid angular positions between the equilibrium positions (V1, V2, R1, R2) of the motor.

16. Apparatus according to claim 15, for a two-phase motor, the apparatus being characterized in that the magnetic sensors (EH) are positioned on the stator at an angular offset of 22·5° relative to the equilibrium positions of the motor.

17. Apparatus according to any one of claims 13 to 16, characterized in that the magnetic sensors are Hall effect cells (EH).

18. Apparatus according to any one of claims 3 to 17, characterized in that the logic circuit (DEC) generates current switching signals (R0-R3) causing current to be applied to the winding(s) of the motor starting from or until an instant (t0') corresponding to a mid angular position between two equilibrium positions of the motor.

19. Apparatus according to any one of claims 3 to 18, comprising an external first portion housing the first sensor means, and an implanted second portion housing the second sensor means, the means for controlling the width of the signal, the logic circuit, and the power circuit, together with flexible cables interconnecting the two portions and constituting a link (B1, B'1) between the first sensor means and the means for controlling the width of the signal.

20. Apparatus according to claim 19, characterized in that during ejection stages, said link (B1, B'1) is used to convey information concerning the beginning of an ejection stage generated in the second portion of the apparatus to the first portion thereof.

21. Apparatus according to claim 20, as dependent on claim 12, the apparatus being characterized in that the second portion thereof includes switching means (T10) controlled by the output of the circuit (BS) connected to the end-of-stroke sensors for the purpose of putting said link at a determined voltage during ejection stages, and in that the first portion of the apparatus includes comparator means (A5) receiving the voltage on said link.

## Patentansprüche

1. Verfahren zum Regeln eines Motors einer peristaltischen Herzprothese mittels elektronischer Umschaltungen, dadurch gekennzeichnet, daß es die folgenden Schritte umfaßt:
- während einer Ausstoßphase wird ein Strom durch den Motor geleitet, welcher dessen Rotation in einer ersten Richtung mit im wesentlichen konstanter Drehzahl veranlaßt, wobei das von dem Motor entwickelte Drehmoment proportional zur Menge des ausgestoßenen Blutes pro Zeiteinheit ist und das vom Motor entwickelte Drehmoment festgestellt wird, um eine Information über den vom menschlichen Körper geforderten Blutausstoß zu erhalten, indem der mittlere, vom Motor aufgenommene Strom gemessen wird und
- während einer Erschlaffungsphase wird ein variabler Strom durch den Motor geleitet, wobei auf die Impulsbreite des durch den Motor geleiteten Stromes eingewirkt wird, um dessen Rotation in der entgegengesetzten Richtung mit einer Drehzahl zu veranlassen, die umso höher ist, je höher der durch den gemessenen mittleren Strom ausgedrückte, geforderte Blutausstoß ist.

2. Verfahren nach Anspruch 1, dadurch gekennzeichnet, daß der mittlere Strom aus einer Vielzahl von Funktionszyklen des Motors gebildet wird.

3. Vorrichtung zum Regeln eines Motors einer peristaltischen Herzprothese mittels elektronischer Umschaltungen, dadurch gekennzeichnet, daß sie Einrichtungen (F1, F2, F3) umfaßt, um folgende Ströme durch den Motor zu leiten:
- während einer Ausstoßphase einen Strom, welcher dessen Rotation in einer ersten Richtung mit im wesentlichen konstanter Drehzahl veranlaßt und
- während einer Erschlaffungsphase einen Strom, welcher dessen Rotation in der entgegengesetzten Richtung mit einer Drehzahl veranlaßt, die umso höher ist, je höher der mittlere durch den Motor fließende, die mittlere Beanspruchung desselben ausdrückende Strom ist
und dadurch, daß sie weiterhin umfaßt:
- erste Meßwertaufnehmer-Einrichtungen (SM, A1 bis A4), um ein elektrisches Signal zu erzeugen, das proportional zum mittleren, durch den Motor fließenden Strom ist,
- zweite Meßwertaufnehmer-Einrichtungen (EH, A12, A13), um elektrische Positionssignale (Q0 bis Q3) zu erzeugen, welche die Winkelposition eines Rotors (RT) des Motors ausdrücken,
- Signalgrößen-Steuereinrichtungen (P10 bis P12, C13), welche derart eingerichtet sind, daß sie
während einer Erschlaffungsphase Steuersignale (SE) erzeugen, deren Größe sich als Funktion des zum mittleren Strom proportionalen elektrischen Signales ändert und
während einer Ausstoßphase Steuersignale erzeugen, deren Größe unveränderlich ist,
- eine logische Schaltung (DEC), welche die Positionsanzeigesignale und die Steuersignale verknüpft, um Stromumschaltsignale (R0 bis R3) zu erzeugen,
- eine Leistungsschaltung (CP), welche die Stromumschaltsignale empfängt und durch mindestens eine Wicklung des Motors einen Strom fließen läßt, dessen zeitlicher Verlauf von diesen Umschaltsignalen abhängt und die Drehzahl des Motors bestimmt.

4. Vorrichtung nach Anspruch 3, dadurch gekennzeichnet, daß die ersten Meßwertaufnehmer-Einrichtungen umfassen:
einen in Reihe zum Stromfluß durch den Motor geschalteten Widerstand (SM) und
Verstärker- und Integrationseinrichtungen (A1 bis A3, DET.S), welche die Spannung an den Anschlüssen des Widerstandes empfangen.

5. Vorrichtung nach Anspruch 4, dadurch gekennzeichnet, daß die Verstärker- und Integrationseinrichtungen einen Integrator (DET.S) aufweisen, dessen Zeitkonstante gleich der Dauer einer Vielzahl von Funktionszyklen der Pumpe ist.

6. Vorrichtung nach Anspruch 5, dadurch gekennzeichnet, daß der Integrator (DET.S) an einen Schwellwertdetektor angeschlossen ist.

7. Vorrichtung nach Anspruch 4, 5 oder 6, dadurch gekennzeichnet, daß die ersten Meßwertaufnehmer-Einrichtungen weiterhin eine Einrichtung (P1) zur manuellen Einstellung aufweisen, um den Anstieg und/oder die Höhe einer monotonen Kurve, welche das elektrische Signal mit dem mittleren Strom verknüpft, einstellen zu können.

8. Vorrichtung nach Anspruch 7, dadurch gekennzeichnet, daß die ersten Meßwertaufnehmer-Einrichtungen weiterhin eine Begrenzereinrichtung (LIM) aufweisen, um die monotone Kurve nach oben zu begrenzen.

9. Vorrichtung nach einem der Ansprüche 4 bis 8, dadurch gekennzeichnet, daß die ersten Meßwertaufnehmer-Einrichtungen dazu vorgesehen sind, den Wert eines elektrischen Stromes zu variieren, der in einer Strombahn (T1, R4) fließen darf.

10. Vorrichtung nach Anspruch 9, dadurch gekennzeichnet, daß die Signalgrößen-Steuereinrichtungen einen Kondensator (C13) aufweisen, welcher derart eingerichtet ist, daß er während der Erschlaffungsphasen durch einen in der Strombahn fließenden Strom (i) geladen oder entladen wird und eine Schwellwerteinrichtung (P12) die Spannung an den Anschlüssen des Kondensators empfängt.

11. Vorrichtung nach Anspruch 10, dadurch gekennzeichnet, daß eine Umschalteinrichtung (I12) vorgesehen ist, welche derart eingerichtet ist, daß sie in Abhängigkeit von der Drehrichtung des Motors, den Kondensator (C13) entweder an die Strombahn oder an einen Festwiderstand (R21) anschließt.

12. Vorrichtung nach Anspruch 11, dadurch gekennzeichnet, daß die Umschalteinrichtung durch eine Schaltung (BS) gesteuert wird, die an Endlagenmelder für ein bewegliches Element der Pumpe angeschlossen ist.

13. Vorrichtung nach einem der Ansprüche 3 bis 12 für einen kollektorlosen Motor, dadurch gekennzeichnet, daß die zweiten Meßwertaufnehmer-Einrichtungen mindestenen einen am Stator des Motors befestigten magnetischen Meßwertaufnehmer (EH) sowie Komparatoreinrichtungen (A12, A13) aufweisen, um logische Positionssignale (SA1, SA2) zu erzeugen, die sich mit dem Vorzeichen der von dem oder den magnetischen Meßwertaufnehmer(n) gelieferten Signale ändern.

14. Vorrichtung nach Anspruch 13 in Verbindung mit Anspruch 11, dadurch gekennzeichnet, daß die Umschalteinrichtungen (I10, I11) zugleich an die zweiten Meßwortaufnehmer-Einrichtungen angeschlossen sind, um den zeitlichen Ablauf der Positionssignale (Q0 bis Q3) bei der Umkehr der Drehrichtung des Motors zu ändern.

15. Vorrichtung nach Anspruch 13 oder 14, dadurch gekennzeichnet, daß der oder die magnetische(n) Meßwertaufnehmer (EH) auf dem Stator derart angeordnet ist (sind), daß die Vorderseiten, welche die Positionssignale begrenzen, in Winkelpositionen angeordnet sind, die sich in der Mitte zwischen den Gleichgewichtspositionen (V1, V2, R1, R2) des Motors befinden.

16. Vorrichtung nach Anspruch 15 für einen Zweiphasen-Motor, dadurch gekennzeichnet, daß die magnetischen Meßwertaufnehmer (EH) auf dem Stator mit einer Winkelversetzung von 22,5° gegenüber den Gleichgewichtspositionen des Motors angeordnet sind.

17. Vorrichtung nach einem der Ansprüche 13 bis 16, dadurch gekennzeichnet, daß die magnetischen Meßwertaufnehmer Hall-Elemente (EH) sind.

18. Vorrichtung nach einem der Ansprüche 3 bis 17, dadurch gekennzeichnet, daß die logische Schaltung (DEC) Stromumschaltsignale (R0 bis R3) erzeugt, welche den Stromfluß in der oder den Wicklung(en) des Motors von dem Zeitpunkt an bzw. bis zu dem Zeitpunkt (t0') auslösen, der einer Mittelposition zwischen den beiden Gleichgewichtspositionen des Motors entspricht.

19. Vorrichtung nach einem der Ansprüche 3 bis 18, mit einem ersten, äußeren Teil, welcher die ersten Meßwertaufnehmer-Einrichtungen schützt und einem zweiten, eingefügten Teil, welcher die zweiten Meßwertaufnehmer-Einrichtungen, die Signalgrößen-Steuereinrichtungen, die logische Schaltung und die Leistungsschaltung schützt, sowie mit biegsamen Verbindungskabeln zwischen den beiden Teilen, welche eine Verbindung (B1, B'1) zwischen den ersten Meßwertaufnehmern und den Signalgrößen-Steuereinrichtungen schaffen.

20. Vorrichtung nach Anspruch 19, dadurch gekennzeichnet, daß die Verbindung (B1, B'1) während der Ausstoßphasen dazu verwendet wird, eine Information über den Beginn der im zweiten Teil der Vorrichtung ausgelösten Ausstoßphase zum ersten Teil der Vorrichtung zu leiten.

21. Vorrichtung nach Anspruch 20 in Verbindung mit Anspruch 12, dadurch gekennzeichnet, daß der zweite Teil der Vorrichtung eine vom Ausgabewert der an die Endlagenmelder angeschlossenen Schaltung (BS) gesteuerte Umschalteinrichtung (T10) aufweist, um der genannten Verbindung während der Ausstoßphasen eine bestimmte Spannung zuzuleiten sowie dadurch, daß der erste Teil der Vorrichtung eine Vergleichereinrichtung (A5) aufweist, welche die Spannung auf dieser Verbindung empfängt.
